# FASCICULE DE BREVET EUROPEEN

(11) **EP 2 814 570 B1**
(45) Date de publication et mention de la délivrance du brevet: **20.04.2016**
(21) Numéro de dépôt: 13708207.9
(22) Date de dépôt: 14.02.2013
(51) Int. Cl.: A61N 5/06

(54) **DISTRIBUTEUR DE PRODUIT FLUIDE**
FLÜSSIGPRODUKTSPENDER
FLUID PRODUCT DISPENSER

(30) Priorité: 17.02.2012 FR 1251482
(43) Date de publication de la demande: 24.12.2014
(73) Titulaire: Aptar France SAS, 27110 Le Neubourg (FR)
(72) Inventeur: DUQUET, Frédéric, 78121 Crespières (FR); MARTINS-REIS, Sandra, 92190 Meudon (FR); MOREAU, Francis, 76300 Sotteville Les Rouen (FR); ROULLET, Florence, 95880 Enghien Les Bains (FR)
(74) Mandataire: CAPRI
(86) Numéro de dépôt international: PCT/FR2013/050294
(87) Numéro de publication internationale: WO 2013/121145

(56) Documents cités:
- US-A1- 2007 185 553
- US-A1- 2007 198 004

## Description

La présente invention concerne un distributeur de produit fluide comprenant un réservoir de produit fluide, un organe de distribution de produit fluide connecté au réservoir, un organe d'actionnement pour actionner l'organe de distribution, un orifice de distribution de produit fluide connecté à l'organe de distribution, et une paroi d'application pour appliquer le produit fluide issu de l'orifice de distribution sur la peau. Ce distributeur, que l'on peut également qualifier d'applicateur, trouve une application privilégiée dans les domaines de la cosmétique, de la pharmacie, et plus généralement dans le domaine des soins et traitements cutanés.

Dans l'art antérieur, il existe déjà de nombreux distributeurs aptes à distribuer du produit fluide, sous forme dosée ou non, afin d'être appliqué sur une surface d'application, tels que la peau, les ongles, des muqueuses, les cheveux, etc. En général, l'organe de distribution est une pompe ou une valve que l'on peut actionner à l'aide d'un poussoir sur lequel on exerce une pression à l'aide d'un ou de plusieurs doigt(s). Le produit fluide en provenance d'un réservoir est mis sous pression dans la chambre de pompe ou de valve avant d'être refoulé à travers un orifice de distribution qui est le plus souvent disposé au niveau d'une paroi d'application destinée à venir en contact de la peau. On se sert de cette paroi d'application pour étaler le produit fluide distribué sur une zone plus ou moins étendue de la peau.

Bien entendu, l'efficacité de ce traitement cutané dépend principalement des caractéristiques du produit fluide appliqué, et de la qualité de l'application. Elle dépend également de la nature et de la qualité de la peau. La présente invention a pour but d'augmenter l'efficacité du traitement cutané en préparant la peau avant l'application du produit fluide. Un autre but est de traiter la peau pendant l'application du produit fluide, et même après l'application du produit fluide.

Pour ce faire, la présente invention propose que le distributeur comprenne en outre au moins une source de rayonnement, telle qu'une diode électroluminescente LED, émettant une lumière monochromatique dans le spectre allant de 400 nm à 700 nm et ayant une action anti-inflammatoire et/ou de stimulation des métabolismes de régénération de la peau.

Ce type de lumière est déjà utilisé en institut pour lutter contre l'inflammation, relancer la synthèse de collagène, améliorer la vascularisation, accélérer la réparation des tissus vergeturés ou cicatriciels et aussi pour réduire les douleurs. Il est ainsi déjà connu de soumettre la peau à des rayonnements de couleur pure ou monochromatique, allant du bleu au rouge. Cependant, c'est surtout le rouge et le proche infrarouge qui permettent de telles performances. La lumière infrarouge est une chaleur par rayonnement dans une longueur d'onde de l'ordre de 700 nm et plus. Cette lumière va chauffer la zone à traiter, stimuler la circulation sanguine, réchauffer les tissus, activer les fibroblastes. Quant à la lumière rouge, elle va principalement stimuler des métabolismes de régénération de la peau en produisant de nouveau collagène : on parle alors de néosynthése de collagène. Pour générer de telles lumières, on peut utiliser des LED ou des lasers basse puissance, souvent désignées par l'acronyme anglais LLT pour Low Level Laser Therapy.

La présente invention prévoit précisément d'associer ou de combiner un distributeur/applicateur classique avec une, voire plusieurs, source(s) de rayonnement de ce type dans le but de traiter la peau avant, pendant et/ou après l'application du produit fluide. On optimise ainsi l'efficacité du produit fluide en agissant sur la qualité de la peau. Les documents US2007/185553 et US2007/198004 décrivent des dispositifs de ce type à usage thérapeutique. L'ergonomie et la forme de ces dispositifs ne sont pas adaptées à une utilisation individuelle à usage cosmétique.

Pour surmonter les problèmes des dispositifs de l'art antérieur, la présente invention propose un distributeur de produit fluide comprenant :
- un réservoir de produit fluide, et
- un corps qui reçoit :
- un organe de distribution de produit fluide connecté au réservoir,
- un organe d'actionnement pour actionner l'organe de distribution,
- une tête de distribution et d'application formant un orifice de distribution de produit fluide connecté à l'organe de distribution, et une paroi d'application pour appliquer le produit fluide issu de l'orifice de distribution sur la peau,
- un module comprenant au moins une source de rayonnement, telle qu'une diode électroluminescente LED, émettant chacune une lumière monochromatique dans le spectre allant de 400 nm à 700 nm et ayant une action anti-inflammatoire et/ou de stimulation des métabolismes de régénération de la peau,
- la tête de distribution et d'application comprenant un logement de source, et en ce que le corps comprend un logement de module dans lequel le module est reçu, ce logement de module communiquant avec le logement de source de sorte que la source de rayonnement s'étend dans le logement de source de la tête.

Le distributeur présente ainsi une forme compacte. Avantageusement, le module est reçu de manière amovible dans le logement de module. Selon un mode de réalisation pratique, le module est solidaire de la tête de distribution et d'application, formant ainsi ensemble une unité solidaire qui est reçue de manière amovible sur et dans le corps. Selon une autre caractéristique, le corps présente une section transversale horizontale de forme oblongue au niveau de laquelle sont disposés l'organe de distribution, l'organe d'actionnement et le logement de module pour le module, l'organe de distribution étant avantageusement disposé entre l'organe d'actionnement et le logement de module, l'organe d'actionnement est avantageusement disposé au niveau d'une partie de plus forte courbure du corps. De préférence, le distributeur présente une forme générale de stylo, avec le corps disposé entre la tête de distribution et d'application et le réservoir.

Le distributeur de l'invention peut ainsi être facilement manipulé tel un stylo par un utilisateur pour une application personnelle, notamment au niveau du visage.

Avantageusement, la source de rayonnement peut émettre une lumière rouge de l'ordre de 660 nm.

Selon une autre caractéristique intéressante, la source de rayonnement peut émettre une lumière au niveau de la paroi d'application. De préférence, la source de rayonnement émet une lumière à travers la paroi d'application. Avantageusement, la paroi d'application est réalisée au moins localement avec un matériau transparent et/ou translucide à la lumière émise par la source de rayonnement, la source émettant une lumière à travers ledit matériau en direction de la peau. De préférence, la paroi d'application guide ou conduit la lumière émise par la source de rayonnement. Ainsi, la paroi d'application remplit une double fonction, à savoir une première fonction de transmission/guidage de la lumière issue de la source de rayonnement et une deuxième fonction plus classique d'application/étalement du produit fluide distribué. Le fait de se servir de la paroi d'application pour guider ou conduire la lumière est une caractéristique particulièrement avantageuse. Un autre avantage inhérent à ce mode de réalisation réside dans le fait que la distance séparant la source lumineuse de la peau est constante et fixe, puisqu'elle est déterminée par la paroi d'application qui elle-même est à une distance déterminée et fixe par rapport à la source lumineuse. On peut ainsi déterminer avec précision le temps de radiation ou d'illumination de la peau en fonction de la puissance de la lumière émise, de la distance qui sépare la source lumineuse de la paroi d'application et des qualités et caractéristiques de diffusion de la paroi d'application. Ainsi, le seul temps d'exposition à la lumière suffit à l'utilisateur pour lui garantir l'effet recherché. D'autre part, le fait d'émettre la lumière à travers la paroi d'application implique forcément que le produit fluide est séparé de la source lumineuse par la paroi d'application, de sorte que la source lumineuse ne peut en aucun cas être souillée avec du produit fluide.

Selon un autre aspect avantageux de l'invention, l'orifice de distribution débouche dans la paroi d'application avec une direction sensiblement parallèle à celle de la lumière émise par la source de rayonnement. Cela implique que la distribution de produit fluide et son application sur la peau peuvent être réalisées directement après la phase d'exposition à la lumière sans manipuler le distributeur autrement qu'en appuyant sur l'organe d'actionnement. En d'autres termes, les phases d'exposition à la lumière et de distribution/application du produit fluide peuvent s'effectuer avec le distributeur maintenu dans une orientation unique et déterminée.

Selon une autre caractéristique particulièrement intéressante de la présente invention, le distributeur comprend en outre des moyens d'alimentation de la source de rayonnement qui sont activés uniquement lorsque la paroi d'application est en contact de la peau. Avantageusement, la paroi d'application fait fonction d'interrupteur pour déclencher l'activation des moyens d'alimentation de la source de rayonnement. Cela signifie que la lumière n'est émise que lorsque la peau est à une distance prédéterminée par la distance séparant la paroi d'application de la source de rayonnement. De ce fait, il est impossible d'irradier volontairement ou involontairement des organes sensibles tels que les yeux. En effet, l'application de la paroi d'application sur un oeil est peut envisageable. On peut utiliser n'importe quelle technique connue de l'état de l'art pour se servir de la paroi d'application en tant qu'interrupteur pour déclencher l'activation de l'alimentation de la source de rayonnement. On peut par exemple prévoir un léger déplacement de la paroi d'application à la manière d'un interrupteur mécanique. On peut également se servir de la paroi d'application comme élément de contact sensible à la chaleur, au toucher, à la présence, etc.

Selon un mode de réalisation pratique, la paroi d'application est formée par une tête de distribution et de transmission comprenant un logement de source pour la réception de la source de rayonnement. Ainsi, la source de rayonnement est parfaitement protégée dans son logement. Elle ne peut être ni abimée, ni souillée.

Selon un autre aspect intéressant de l'invention, l'organe d'actionnement est mobile selon une direction d'actionnement qui est sensiblement perpendiculaire à une direction de distribution du produit fluide à travers l'orifice de distribution. Ainsi, on peut saisir et se servir du distributeur à la manière d'un stylo en appuyant latéralement à l'aide de l'index sur l'organe d'actionnement, ce qui permet de maintenir le distributeur avec une grande précision.

Selon un autre aspect pratique de l'invention, la source de rayonnement fait partie d'un module comprenant en outre une circuiterie électronique, des moyens d'alimentation, des moyens d'activation des moyens d'alimentation, et un minuteur. Les moyens d'activation qui vont déclencher les moyens d'alimentation peuvent par exemple comprendre un capteur sensible à la pression, au contact, à la présence, à la chaleur ou encore sous la forme d'un compacteur électrique.

Selon un mode de réalisation pratique, le réservoir se présente sous la forme d'une cartouche amovible et remplaçable, qui est disposée dans un étui connecté de manière amovible au corps. Le distributeur comprend alors deux sous-ensembles distincts, à savoir un sous-ensemble inférieur constitué par le réservoir et l'étui et un sous-ensemble supérieur constitué par le corps, l'organe de distribution, l'organe d'actionnement, la tête de distribution, le module et optionnellement un capot qui vient coiffer la paroi d'application.

L'esprit de l'invention réside dans le fait d'intégrer dans un même distributeur une ou plusieurs source(s) de rayonnement capable(s) de traiter la peau et un applicateur capable d'étaler un produit fluide sur la peau. La configuration compacte et la forme ergonomique comparable à celles d'un stylo permet une utilisation facile.

L'invention sera maintenant décrite plus amplement en référence aux dessins joints donnant à titre d'exemple non limitatif un mode de réalisation de l'invention.

Sur les figures :
La figure 1 est une vue en coupe transversale verticale à travers un distributeur de produit fluide selon l'invention,
La figure 2 est une vue en perspective éclatée du distributeur de la figure 1,
La figure 3 est une vue fortement agrandie de la partie supérieure entourée du distributeur de la figure 1 qui montre de manière schématique certains composants du module, et
La figure 4 est une vue semblable à celle de la figure 3 pour une variante de réalisation à l'état démonté.

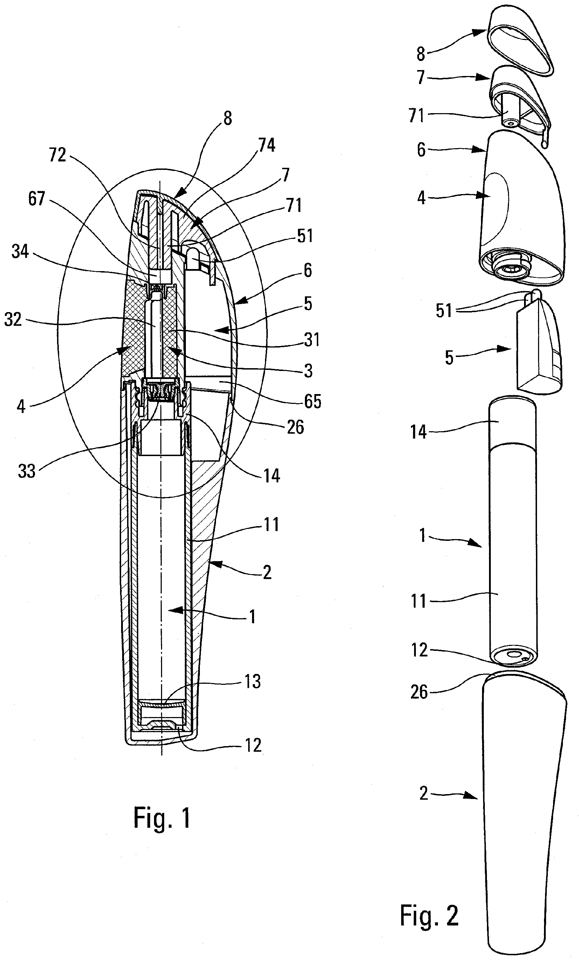

On se référera indifféremment à l'ensemble des figures 1 à 3 pour décrire en détail la structure et le fonctionnement d'un distributeur selon un mode de réalisation non limitatif de l'invention.

Le distributeur comprend un réservoir de produit fluide 1, un étui 2 dans lequel le réservoir 1 est reçu, un organe de distribution 3 qui est ici une pompe, un organe d'actionnement 4 pour actionner la pompe 3 et qui est ici intégré à la pompe, un module 5 doté d'une (voire plusieurs) source de rayonnement 51, un corps 6 qui reçoit la pompe 3, l'organe d'actionnement 4 et le module 5, et enfin une tête de distribution 7 qui est montée sur le corps 6. Optionnellement, le distributeur peut encore comprendre un capot de protection 8 qui coiffe la tête de distribution 7. Tous les éléments constitutifs du distributeur, à l'exception du module 5, peuvent être réalisés par injection moulage de matière plastique. Le distributeur est représenté environ à l'échelle 1 sur la figure 1. On peut remarquer que le distributeur n'est pas symétrique de révolution, mais plutôt bombé unilatéralement du côté du module 5. Le distributeur est destiné à être saisi à la manière d'un stylo avec la partie bombée orientée vers la paume de la main et l'organe d'actionnement 4 destiné à venir en contact avec l'index. On peut remarquer sur la figure 2 que la partie haute de l'étui 2 et le corps 6 présentent une section transversale horizontale de forme allongée, oblongue ou ovoïdale. L'organe d'actionnement 4 est avantageusement disposé au niveau d'une partie de plus forte courbure du corps. Le distributeur présente ainsi une configuration plus étroite dans un sens que dans l'autre ce qui améliore sa préhension à l'aide d'une main à la manière d'un stylo. Intuitivement, l'utilisateur va saisir le distributeur de manière à disposer l'organe d'actionnement 4 sous son index. Le distributeur va principalement être maintenu entre le pouce et le majeur au niveau du corps 6, de part et d'autre du module 5 et de l'organe d'actionnement 4.

Le réservoir de produit fluide 1 peut être réalisé avec une section circulaire cylindrique et comprendre un fût 11 à l'intérieur duquel se déplace un piston suiveur 13. Pour empêcher toute dépression dans le fût sous le piston suiveur 13, le fût est doté d'un trou d'évent 12. A son extrémité supérieure, le fût 11 est pourvu d'une bague de fixation 14 pourvue d'un filetage interne 15. De manière tout à fait classique, le piston suiveur 13 va se déplacer à l'intérieur du fût 11 à mesure que du produit fluide en est extrait. Ainsi, il ne pénètre jamais d'air à l'intérieur du réservoir 1. Il s'agit là d'un mode de réalisation particulier non limitatif : en effet, n'importe quel autre réservoir avec ou sans reprise d'air peut être utilisé dans le cadre de la présente invention.

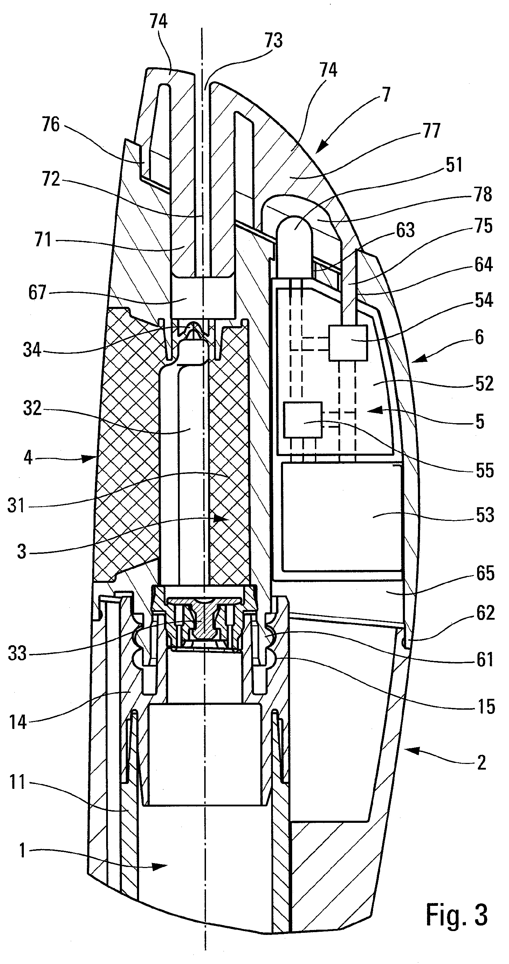

Le réservoir 1 est disposé à l'intérieur de l'étui 2 qui a pour principale fonction de protéger et masquer le réservoir 1. Comme susmentionné, on peut remarquer que l'étui 2 présente une forme géométrique complexe avec une partie haute de section transversale horizontale oblongue ou ovoïdale. Toutefois, l'étui 1 définit intérieurement un logement de réception axiale pour le réservoir 1. On peut ainsi dire que l'étui 2 forme une excroissance unilatérale qui s'étend d'un seul côté du réservoir 1, comme on peut le voir sur les figures 1 et 3. L'étui 2 comprend un bord d'ouverture 26 destiné à venir en prise avec le corps 6 comme on le verra ci-après.

L'organe de distribution 3, qui est ici une pompe, comprend un corps de pompe 31 définissant une chambre de pompe 32 qui communique en partie basse avec le réservoir 1 à travers un clapet d'entrée 33 et qui est pourvu en partie haute d'un clapet de sortie 34. La pompe 3 est ici d'un type particulier, dit pompe à membrane, étant donné que l'organe d'actionnement 4 se présente sous la forme d'une paroi d'actionnement souple qui fait avantageusement partie intégrante du corps de pompe 3. En appuyant à l'aide d'un doigt, par exemple l'index, sur la paroi déformable 4, on réduit le volume utile de la chambre de pompe 32, ce qui met le produit fluide qu'il contient sous pression, ferme le clapet d'entrée 33 et ouvre le clapet de sortie 34 à travers lequel le produit sous pression est refoulé. Il s'agit là d'un type et d'un fonctionnement connus pour une pompe dite à membrane. La structure particulière de la pompe 3 n'est pas critique pour la présente invention : on peut mettre en oeuvre un autre type de pompe sans sortir du cadre de l'invention.

La pompe 3 avec son organe d'actionnement 4 sont intégrés dans le corps 6 qui peut par exemple être surmoulé sur la pompe 3 et son organe d'actionnement 4. Le corps 6 forme une douille d'assemblage 62 qui est filetée extérieurement de sorte que le filetage interne 15 de la bague 14 peut venir en prise pour raccorder l'ouverture du réservoir 1 à l'entrée de la pompe ou se trouve le clapet d'entrée 33. Un autre type d'assemblage, par exemple par encliquetage ou à baïonnette, peut également être utilisé pour raccorder le réservoir 1 au corps 6, ou plus généralement à l'entrée de la pompe 3. Il est préférable dans le cadre de l'invention que le réservoir 1 soit connecté de manière amovible au corps 6 de sorte qu'il peut être remplacé à la manière d'une cartouche. On peut à cet effet remarquer que l'étui 2 est raccordé au niveau de son bord d'ouverture 26 de manière encliquetée avec une bride 62 formée à l'extrémité inférieure du corps 6. Pour remplacer le réservoir 1 une fois vide, il suffit de désencliqueter l'étui 2 du corps 6 et de dévisser le réservoir 1 de la douille filetée 61 du corps 6. On peut par exemple prévoir que l'ouverture du réservoir 1 est obturée initialement par un opercule qui est percé par un organe quelconque solidaire du corps 6 ou du clapet d'entrée 33 lors du vissage du réservoir 1 sur la douille filetée 61. Dès lors, on peut définir deux sous-ensembles, à savoir un sous-ensemble inférieur constitué par le réservoir 1 et l'étui 2, et un sous-ensemble supérieur constitué par le corps 6 et tous les éléments constitutifs qui sont montés dessus.

A la sortie du clapet de sortie 34, le corps 6 forme une cheminée de sortie 67 qui mène jusqu'à l'orifice de distribution 73 comme on le verra ci-après. Le corps 6 définit également un logement de module 65 à l'intérieur duquel est reçu le module 5. Le corps 6 forme une première fenêtre 63 à travers laquelle s'étend la source lumineuse 51, par exemple sous la forme d'une ou de plusieurs diodes électroluminescentes LED. Le corps 6 définit une seconde fenêtre 64 dont la fonction sera donnée ci-après.

La tête de distribution et d'application 7 est une pièce asymétrique qui peut être réalisée par injection moulage de matière plastique transparente ou translucide. La tête 7 comprend un tube de sortie 71 définissant intérieurement un canal de sortie 72 reliant la cheminée de sortie 67 à l'orifice de distribution 73. Le tube de sortie 71 peut par exemple être emmanché en force à l'intérieur de la cheminée 67. La tête 7 comprend également une paroi d'application 74 dans laquelle débouche l'orifice de distribution 73. Dans la forme de réalisation des figures, la paroi d'application 74 s'étend majoritairement sur le côté gauche de l'orifice de distribution 73 en définissant une pente courbe. On peut remarquer que la paroi d'application 74 présente une surépaisseur qui va servir de guide d'onde de lumière 77, comme on le verra ci-après. La surépaisseur formant guide d'onde 77 définit un logement de source 78 pour la réception de la ou les source(s) de rayonnement 51. La tête 7 comprend également une lèvre de stabilisation périphérique 76 pour assurer le maintien fixe stable et définitif de la tête 7 sur le corps 6. La tête 7 comprend également une broche ou patte de transmission 75 qui s'étend à travers la deuxième fenêtre 64 pour pénétrer éventuellement à l'intérieur du logement de module 65. La paroi d'application 74 est réalisée au moins partiellement ou localement avec un matériau transparent ou translucide à la lumière du visible et aux infrarouges. On peut par exemple utiliser des copolyesters ou des styrèniques pour réaliser la paroi d'application, et même la tête 7 dans son ensemble.

On comprend déjà qu'il est possible de distribuer du produit fluide à travers l'orifice de distribution 73 au niveau de la surface d'application 74 en actionnant l'organe d'actionnement 4 de l'organe de distribution 3 qui permet de prélever du produit fluide du réservoir 1, de le mettre sous pression dans la chambre 32 et de le refouler à travers le clapet de sortie 34, la cheminée 67 et le conduit de sortie 72 jusqu'à l'orifice de distribution 73. Une fois le produit distribué présent sur la paroi d'application 74, l'utilisateur peut l'appliquer et l'étaler sur une surface d'application, telle que la peau. Le produit fluide distribué est de préférence un produit visqueux, tels que de la crème, un gel, une pommade, etc.

Le module 5 est un module électronique doté d'une ou de plusieurs source(s) de rayonnement 51 sous la forme d'une ou de plusieurs diode(s) électroluminescente(s) LED par exemple. Chaque LED émet une lumière monochromatique. On peut par exemple prévoir une LED bleue et/ou une LED rouge. Le module 5 peut se présenter sous la forme d'un petit boitier coiffé d'une ou de deux LED(s). Le module comprend des moyens d'alimentation 53, par exemple sous la forme d'une batterie, une circuiterie électronique 52, par exemple sous la forme d'une petite plaque de circuit imprimée, des moyens d'activation 54 pour déclencher l'alimentation de la source 51 par les moyens d'alimentation 53, et un temporiseur ou minuteur 55 qui détermine le temps d'alimentation de la source lumineuse 51. En d'autres temps, les moyens d'activation 54 déterminent le début de la phase d'alimentation de la source lumineuse 51 et le minuteur 55 détermine la fin de la phase d'alimentation de la source 51. Bien entendu, le module 5 peut intégrer encore d'autres composants électroniques aptes à remplir d'autres fonctions. Le module 5 est disposé et maintenu à l'intérieur du logement de module 65 formé par le corps 6. Dans cette position montée, la source lumineuse 51 s'étend à travers la première fenêtre 63 du module 6 à l'intérieur d'un logement de source 78 défini à l'intérieur de la tête de distribution 7. Ce logement de source 78 est en grande partie formé par la paroi d'application 74 réalisée en un matériau qui est transparent ou translucide à la lumière émise par la source lumineuse source de rayonnement 51. A cet effet, la lumière émise par la source 51 peut être dans un spectre de 400 nm à 700 nm allant de l'ultraviolet à l'infrarouge. On peut par exemple utiliser une lumière rouge dont la longueur d'onde est de l'ordre de 660 nm. La peau exposée à une telle lumière va entre autres subir une stimulation des métabolismes de régénération de la peau et produire du collagène. Cette lumière va ainsi être émise dans le logement de source 78, être guidée par la surépaisseur formant guide d'onde 77, puis sortir de la paroi d'application 74 pour atteindre la peau qui est en contact de la surface d'application 74. De préférence, le matériau transparent ou translucide constitutif de la paroi d'application 74 permet de diffuser la lumière qui la traverse dans toutes les directions de manière à homogénéiser la répartition lumineuse à la surface de la peau. Lorsque la tête 7 est entièrement réalisée avec un tel matériau diffuseur, tous les éléments constitutifs de la tête peuvent servir de guides d'ondes et de transmission, y compris le tube de sortie 71.

D'autre part, la broche de transmission 75 s'étend de la surface d'application 74 à travers la seconde fenêtre 64 jusque dans le logement de module 65 de manière à coopérer directement ou indirectement avec les moyens d'activation 54. La broche de transmission 75 sert de guide, conducteur ou transmetteur permettant d'acheminer une information au module 5 en provenance de la paroi d'application 74. On peut par exemple prévoir que la broche 75 est susceptible de transmettre aux moyens d'activation 54 une force de poussée exercée sur la surface d'application 74. Dans ce cas, les moyens d'activation 54 peuvent comprendre un détecteur de poussée ou plus simplement un contacteur électrique sensible à la poussée. Dès que les moyens d'activation 54 perçoivent une pression, il déclenche l'alimentation électrique de la source lumineuse 51. On peut également prévoir que la broche de transmission 75 soit adaptée à transmettre de la chaleur, un contact avec un autre élément ou l'absence de lumière. On peut également prévoir d'autres moyens de transmission, comme des conducteurs électriques, thermiques ou optiques. Dans ces cas, on peut prévoir que les moyens d'activation 54 comprennent un capteur de chaleur, de présence ou d'absence de lumière. On peut également prévoir un contacteur électronique au niveau de la paroi d'application 74. Il existe de nombreuses techniques dans l'état de l'art pour transmettre une information à des moyens d'activation. Dans le cadre de la présente invention, le but recherché est l'activation de la source lumineuse 51 uniquement lorsque la paroi d'application 74 est en contact avec la peau. Ce contact avec la peau crée une pression, une transmission de chaleur, un courant électrique et/ou un obscurcissement de la paroi d'application : un ou plusieurs de ces paramètre(s) peuvent être transmis par l'intermédiaire de la broche de transmission 75 au module 5 pour qu'elle déclenche l'alimentation de la source lumineuse 51. On évite ainsi tout déclenchement accidentel ou néfaste pour la santé. Après activation, la source de rayonnement 51 va rester alimentée pendant un temps prédéterminé par le minuteur 55 afin d'obtenir un traitement adéquat de la zone de peau qui va ensuite recevoir l'application du produit fluide. Cependant, l'alimentation doit être coupée, interrompant ainsi le minuteur, dès que la paroi n'est plus en contact de la peau pour éviter une irradiation accidentelle. On peut aussi prévoir que l'écoulement de temps du minuteur est uniquement suspendu, et non pas réinitialisé, lorsque l'applicateur est brièvement décollé de la peau.

Il faut remarquer que la distance séparant la source lumineuse 51 de la peau est déterminée par la surface d'application 74 contre laquelle la peau va venir en contact. On peut ainsi déterminer avec précision la puissance de la source lumineuse 51, sa distance à la surface d'application 74 et son temps d'activation pour obtenir le traitement cutané souhaité. D'autre part, étant donné que la source lumineuse 51 est disposée en dessous de la paroi d'application 74, elle ne peut pas venir en contact direct du produit fluide : elle ne peut ainsi pas être souillée par ce dernier.

On peut également noter que le module 5 peut aisément être extrait de son logement 65 pour être remplacé par un nouveau module, en cas de défaillance ou de dysfonctionnement. Le module 5 se présente alors sous la forme d'une simple pièce de rechange. Il est également possible d'extraire le module 5 de son logement 65 simplement pour remplacer ou charger les moyens d'alimentation 53.

Etant donné que la surface d'application 54 peut aisément être nettoyée, on garantit une exposition constante et reproductible de la peau à la lumière de la source de rayonnement 51.

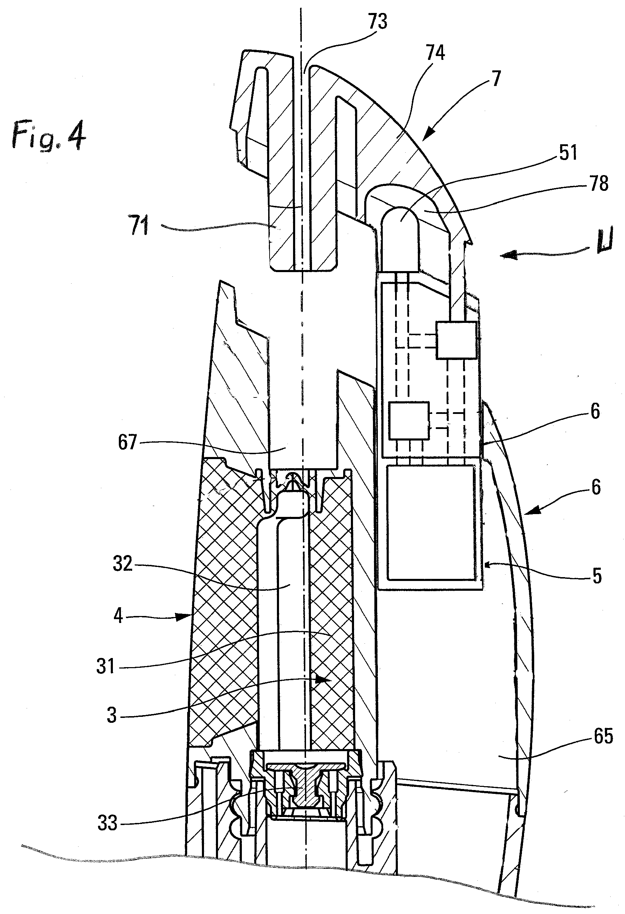

La figure 4 représente une variante de réalisation dans laquelle la tête 7 et le module 5 forment ensemble une unité solidaire U qui est montée sur le corps 6. L'unité U est représentée en cours de montage ou de démontage sur la figure 4. Le module est déjà inséré à travers une fenêtre unique 66 qui permet d'accéder dans le logement de module 65. Le tube de sortie 71 est aligné avec la cheminée de sortie 67.

Grâce à l'invention, un distributeur/applicateur classique voit son efficacité considérablement améliorée de par le traitement préalable, simultané et/ou postérieur de la zone de la peau au moyen d'une lumière appropriée.

## Revendications

1. Distributeur de produit fluide comprenant :
- un réservoir de produit fluide (1), et
- un corps (6) qui reçoit :
- un organe de distribution de produit fluide (3) connecté au réservoir (1),
- un organe d'actionnement (4) pour actionner l'organe de distribution (3),
- une tête de distribution et d'application (7) formant un orifice de distribution de produit fluide (73) connecté à l'organe de distribution (3), et une paroi d'application (74) pour appliquer le produit fluide issu de l'orifice de distribution (73) sur la peau,
- un module (5) comprenant au moins une source de rayonnement (51), telle qu'une diode électroluminescente LED, émettant chacune une lumière monochromatique dans le spectre allant de 400 nm à 700 nm, le module (5) comprenant en outre une circuiterie électronique (52), des moyens d'alimentation (53), des moyens d'activation (54) des moyens d'alimentation, et un minuteur (55), reçus dans le logement de module (65) du corps (6),
la tête de distribution et d'application (7) comprenant un logement de source (78), et le corps (6) comprenant un logement de module (65) dans lequel le module (5) est reçu, ce logement de module (65) communiquant avec le logement de source (78) de sorte que la source de rayonnement (51) s'étend dans le logement de source (78) de la tête (7).

2. Distributeur selon la revendication 1, dans lequel le module (5) est reçu de manière amovible dans le logement de module (65).

3. Distributeur selon la revendication 1 ou 2, dans lequel le module (15) est solidaire de la tête de distribution et d'application (7), formant ainsi ensemble une unité solidaire (U) qui est reçue de manière amovible sur et dans le corps (6).

4. Distributeur selon l'une quelconque des revendications précédentes, dans lequel le corps (6) présente une section transversale horizontale de forme oblongue au niveau de laquelle sont disposés l'organe de distribution (3), l'organe d'actionnement (4) et le logement de module (65) pour le module (5), l'organe de distribution (3) étant avantageusement disposé entre l'organe d'actionnement (4) et le logement de module (65), l'organe d'actionnement étant disposé au niveau d'une partie de plus forte courbure du corps (6).

5. Distributeur selon l'une quelconque des revendications précédentes, présentant une forme générale de stylo, avec le corps (6) disposé entre la tête de distribution et d'application (7) et le réservoir (1).

6. Distributeur selon la revendication 1, dans lequel la source de rayonnement (51) émet une lumière rouge de l'ordre de 660 nm.

7. Distributeur selon l'une quelconque des revendications précédentes, dans lequel la source de rayonnement (51) émet une lumière à travers la paroi d'application (74), la paroi d'application (74) étant réalisée au moins localement avec un matériau transparent et/ou translucide à la lumière émise par la source de rayonnement (51), la source émettant une lumière à travers ledit matériau en direction de la peau.

8. Distributeur selon l'une quelconque des revendications précédentes, dans lequel l'orifice de distribution (73) débouche dans la paroi d'application (74) avec une direction sensiblement parallèle à celle de la lumière émise par la source de rayonnement (51).

9. Distributeur selon l'une quelconque des revendications précédentes, comprenant en outre des moyens d'alimentation (55) de la source de rayonnement (51) qui sont activés uniquement lorsque la paroi d'application (74) est en contact de la peau.

10. Distributeur selon la revendication 8, dans lequel la paroi d'application (74) fait fonction d'interrupteur pour déclencher l'activation des moyens d'alimentation (53) de la source de rayonnement (51).

11. Distributeur selon l'une quelconque des revendications précédentes, dans lequel l'organe d'actionnement (4) est mobile selon une direction d'actionnement qui est sensiblement perpendiculaire à une direction de distribution du produit fluide à travers l'orifice de distribution (73).

12. Distributeur selon l'une quelconque des revendications précédentes, dans lequel le réservoir (1) se présente sous la forme d'une cartouche amovible et remplaçable, qui est disposée dans un étui (2) connecté de manière amovible au corps (6).

## Patentansprüche

1. Spender für ein fluides Produkt, aufweisend:
- einen Behälter für fluides Produkt (1) und
- einen Körper (6), der aufnimmt:
- ein Ausgabeelement für ein fluides Produkt (3), das mit dem Behälter (1) verbunden ist,
- ein Betätigungselement (4) zum Betätigen des Ausgabeelements (3),
- einen Ausgabe- und Aufbringungskopf (7), der eine Ausgabeöffnung für ein fluides Produkt (73) bildet, die mit dem Ausgabeelement (3) verbunden ist, und eine Aufbringungswand (74) zum Aufbringen des aus der Ausgabeöffnung (73) ausgetretenen fluiden Produktes auf die Haut,
- ein Modul (5), aufweisend mindestens eine Strahlungsquelle (51), wie eine Lumineszenzdiode LED, die jeweils ein einfarbiges Licht im Spektrum von 400 nm bis 700 nm ausstrahlt, wobei das Modul (5) des Weiteren eine elektronische Schaltung (52), Versorgungsmittel (53), Aktivierungsmittel (54) für die Versorgungsmittel und einen Zeitgeber (55) aufweist, die in der Modulaufnahme (65) des Körpers (6) aufgenommen sind,
wobei der Ausgabe- und Aufbringungskopf (7) eine Quellenaufnahme (78) aufweist und der Körper (6) eine Modulaufnahme (65) aufweist, in der das Modul (5) aufgenommen ist, wobei diese Modulaufnahme (65) mit der Quellenaufnahme (78) derart kommuniziert, dass sich die Strahlungsquelle (51) in die Quellenaufnahme (78) des Kopfes (7) erstreckt.

2. Spender nach Anspruch 1, wobei das Modul (5) lösbar in der Modulaufnahme (65) aufgenommen ist.

3. Spender nach Anspruch 1 oder 2, wobei das Modul (15) mit dem Ausgabe- und Aufbringungskopf (7) einstückig ausgebildet ist und so zusammen eine einstückige Einheit (U) bildet, die lösbar auf und in dem Körper (6) aufgenommen ist.

4. Spender nach einem der vorhergehenden Ansprüche, wobei der Körper (6) einen horizontalen Querschnitt von länglicher Form bildet, an dem das Ausgabeelement (3), das Betätigungselement (4) und die Modulaufnahme (65) für das Modul (5) angeordnet sind, wobei das Ausgabeelement (3) vorteilhafterweise zwischen dem Betätigungselement (4) und der Modulaufnahme (65) angeordnet ist, wobei das Betätigungselement an einem Teil des Körpers (6) mit einer stärkeren Krümmung angeordnet ist.

5. Spender nach einem der vorhergehenden Ansprüche, der eine im Allgemeinen stiftartige Form darstellt, wobei der Körper (6) zwischen dem Ausgabe- und Aufbringungskopf (7) und dem Behälter (1) angeordnet ist.

6. Spender nach Anspruch 1, wobei die Strahlungsquelle (51) ein Rotlicht im Bereich von 660 nm ausgibt.

7. Spender nach einem der vorhergehenden Ansprüche, wobei die Strahlungsquelle (51) ein Licht durch die Aufbringungswand (74) ausgibt, wobei die Aufbringungswand (74) zumindest lokal mit einem für das von der Strahlungsquelle (51) ausgegebene Licht transparenten und/oder durchscheinenden Material ausgebildet ist, wobei die Quelle ein Licht durch das Material in Richtung zur Haut ausgibt.

8. Spender nach einem der vorhergehenden Ansprüche, wobei die Ausgabeöffnung (73) in der Aufbringungswand (74) in einer Richtung im Wesentlichen parallel zu derjenigen des von der Strahlungsquelle (51) ausgegebenen Lichts mündet.

9. Spender nach einem der vorhergehenden Ansprüche, des Weiteren aufweisend Versorgungsmittel (55) für die Strahlungsquelle (51), die nur aktiviert werden, wenn die Aufbringungswand (74) mit der Haut in Kontakt ist.

10. Spender nach Anspruch 8, wobei die Aufbringungswand (74) als Unterbrecher dient, um die Aktivierung der Versorgungsmittel (53) der Strahlungsquelle (51) auszulösen.

11. Spender nach einem der vorhergehenden Ansprüche, wobei das Betätigungselement (4) entlang einer Betätigungsrichtung beweglich ist, die zu einer Ausgaberichtung des fluiden Produkts durch die Ausgabeöffnung (73) im Wesentlichen senkrecht ist.

12. Spender nach einem der vorhergehenden Ansprüche, wobei der Behälter (1) die Form einer lösbaren und ersetzbaren Kartusche aufweist, die in einer Hülse (2) angeordnet ist, die lösbar mit dem Körper (6) verbunden ist.

## Claims

1. A fluid dispenser comprising:
· a fluid reservoir (1); and
· a body (6) that receives:
· a fluid dispenser member (3) that is connected to the reservoir (1);
· an actuator member (4) for actuating the dispenser member (3);
· a dispenser and applicator head (7) forming a fluid dispenser orifice (73) that is connected to the dispenser member (3), and an application wall (74) for applying the fluid coming from the dispenser orifice (73) onto the skin; and
· a module (5) including at least one source of radiation (51), such as an LED, each emitting monochromatic light in the spectrum lying in the range 400 nm to 700 nm, the module (5) further including electronic circuitry (52), power supply means (53), activator means (54) for activating the power supply means, and a timer (55), received in the module housing (65) of the body (6),
the dispenser and applicator head (7) including a source housing (78), and the body (6) including a module housing (65) in which the module (5) is received, the module housing (65) communicating with the source housing (78) so that the source of radiation (51) extends into the source housing (78) of the head (7).

2. A dispenser according to claim 1, wherein the module (5) is received in removable manner in the module housing (65).

3. A dispenser according to claim 1 or claim 2, wherein the module (5) is secured to the dispenser and applicator head (7), thereby together forming an integral unit (U) that is received in removable manner on and in the body (6).

4. A dispenser according to any preceding claim, wherein the body (6) presents a horizontal cross-section of shape that is oblong, where the dispenser member (3), the actuator member (4), and the module housing (65) for the module (5) are arranged, the dispenser member (3) advantageously being arranged between the actuator member (4) and the module housing (65), the actuator member being arranged at a portion of the body (6) that is more curved.

5. A dispenser according to any preceding claim, presenting the general shape of a pen, with the body (6) arranged between the dispenser and applicator head (7) and the reservoir (1).

6. A dispenser according to claim 1, wherein the source of radiation (51) emits red light at about 660 nm.

7. A dispenser according to any preceding claim, wherein the source of radiation (51) emits light through the application wall (74) the application wall (74) being made, at least locally, out of a material that is transparent and/or translucent to the light emitted by the source of radiation (51), the source emitting light through said material towards the skin.

8. A dispenser according to any preceding claim, wherein the dispenser orifice (73) opens out to the application wall (74) with a direction that is substantially parallel to the direction of the light emitted by the source of radiation (51).

9. A dispenser according to any preceding claim, further including power supply means (55) for powering the source of radiation (51), which means are activated only when the application wall (74) is in contact with the skin.

10. A dispenser according to claim 8, wherein the application wall (74) causes a switch to operate so as to trigger the activation of the power supply means (53) for powering the source of radiation (51).

11. A dispenser according to any preceding claim, wherein the actuator member (4) is movable in an actuation direction that is substantially perpendicular to a direction for dispensing the fluid through the dispenser orifice (73).

12. A dispenser according to any preceding claim, wherein the reservoir (1) is in the form of a removable and replaceable cartridge that is arranged in a casing (2) that is connected in removable manner to the body (6).
